# DEMANDE DE BREVET EUROPEEN

(11) **EP 0 847 985 A1**
(43) Date de publication de la demande: **17.06.1998**
(21) Numéro de dépôt: 97402614.8
(22) Date de dépôt: 03.11.1997
(51) Int. Cl.: C07C 213/00

(54) **Procédé de préparation de dérivés ammoniums quaternaires hydroxypropyles à fonction ester d'acide gras**

(30) Priorité: 11.12.1996 FR 9615223
(71) Demandeur: L'OREAL, 75008 Paris (FR)
(72) Inventeur: Philippe, Michel, 91320 Wissous (FR); Campos, Alain, 77290 Mitry-Mory (FR)
(74) Mandataire: Dodin, Catherine

(57) **Abrégé**

La présente demande concerne un procédé de préparation d'ammoniums quaternaires hydroxypropylés à fonction ester d'acide gras de formule (I) suivante : 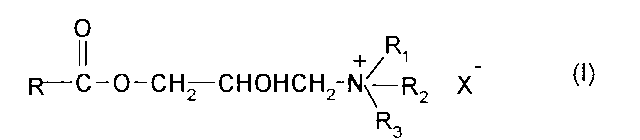 dans laquelle R désigne une chaîne alkyle en C₇-C₃₅;
R₁, R₂ et R₃, identiques ou différents, désignent une chaîne alkyle en C₁-C₁₈ ;
X⁻ désigne un halogénure ou un anion choisi dans le groupe constitué par : 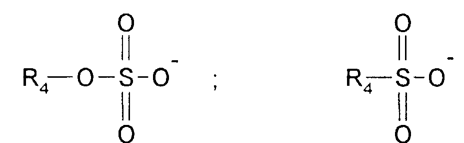 et 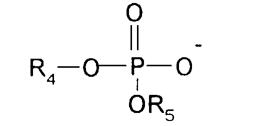 dans lesquels R₄ et R₅, identiques ou différents, désignent une chaîne alkyle en C₁-C₈ ; ledit procédé consistant en une seule étape à faire réagir, dans un solvant alcoolique à reflux, un sel d'acide carboxylique RCOOH ou un acide carboxylique en catalyse basique avec un composé de formule (II) suivante : 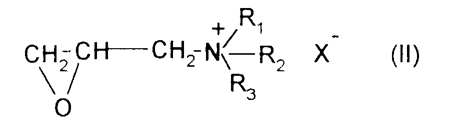 où R, R₁, R₂, R₃ et X⁻ ont les mêmes significations indiquées ci-dessus.

## Description

La présente invention a trait à un nouveau procédé de préparation d'ammoniums quaternaires hydroxypropylés à fonction ester d'acide gras.

On utilise certains composés ammoniums quaternaires hydroxypropylés à fonction ester d'acide gras en cosmétique dans des produits capillaires, comme agents de traitement conférant aux cheveux un effet brillant, un toucher doux, et facilitant le peignage et le démêlage des cheveux. Ils sont décrits dans les brevets FR 1 313 143 et US 3 872 138.

Selon le brevet FR 1 313 143, ces composés sont préparés selon un procédé de synthèse en deux étapes consistant :
(1°) à faire réagir un sel alcalin d'acide carboxylique aliphatique gras avec une épihalohydrine, en particulier l'épichlorhydrine, et une amine secondaire dans un solvant alcoolique (isopropanol ou tertio butanol) et
(2°) à séparer l'halogénure de métal alcalin formé et à effectuer une quaternisation de l'aminoester formé dans la première étape par un agent alkylant tel que le sulfate de diméthyle.

Cette synthèse présente l'inconvénient d'être longue et coûteuse , de manipuler l'épichlorhydrine très toxique et de mise en oeuvre délicate et nécessite l'emploi d'agents alkylants hautement toxiques.

Selon le brevet US 3 872 138, ces mêmes composés sont préparés selon un procédé en deux étapes consistant à faire réagir une amine tertiaire avec un acide carboxylique dans des quantités stoechiométriques pour neutraliser l'acide par l'amine puis à faire réagir le sel résultant avec de l'épichlorhydrine dans un solvant du type benzène à une température de 50 à 150°C pendant une durée en général de 15 heures. Cette synthèse présente l'inconvénient d'être également longue et coûteuse, de manipuler l'épichlorhydrine et nécessite l'emploi de solvants très toxiques.

La Demanderesse a découvert de manière surprenante un nouveau procédé permettant d'obtenir des composés ammoniums quaternaires hydroxypropylés à fonction ester d'acide gras en une seule étape dans des conditions de mise en oeuvre plus faciles, plus rapides et plus sécurisantes sans utiliser d'épihalohydrine, d'agent alkylant ni de solvant à haute toxicité.

Le procédé conforme à la présente invention a pour but de préparer des dérivés ammoniums quaternaires hydroxypropylés à fonction ester d'acide gras de formule générale : 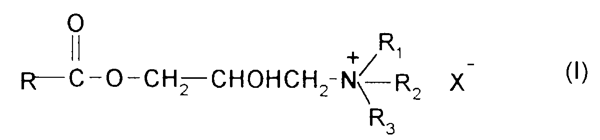 dans laquelle
R désigne une chaîne alkyle, ramifiée ou linéaire, saturée ou insaturée en C₇-C₃₅ ;
R₁, R₂ et R₃, identiques ou différents, désignent une chaîne alkyle, ramifiée ou linéaire, saturée ou insaturée en C₁-C₁₈ ;
X⁻ désigne un halogénure(préférentiellement le chlore) ou un anion choisi dans le groupe constitué par : 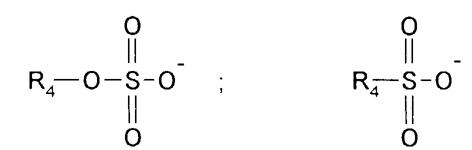 et 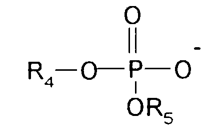
dans lesquels R₄ et R₅, identiques ou différents, désignent une chaîne alkyle, ramifiée ou linéaire, saturée ou insaturée en C₁-C₈.

On prépare de façon préférentielle, les composés de formule(I) où R₁, R₂ et R₃ désignent simultanément un radical méthyle et X⁻ désigne l'ion chlorure.

Ledit procédé est caractérisé par le fait qu'il consiste, en une seule étape, à faire réagir, dans un solvant alcoolique à reflux, un sel d'acide carboxylique aliphatique gras RCOOH ou un acide carboxylique aliphatique en catalyse basique avec un composé de formule (Il) suivante : 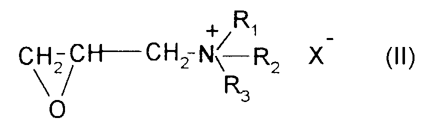 où R, R₁, R₂, R₃ et X⁻ ont les mêmes significations indiquées ci-dessus.

Le sel d'acide carboxylique est de préférence un sel de métal alcalin tel que de sodium ou de potassium ou un sel d'alcalino-terreux. Les sels de métal alcalin sont particulièrement préférés.

La catalyse basique est de préférence réalisée avec la soude, la potasse ou la triéthylamine.

Le solvant alcoolique utilisé est de préférence choisi parmi les alcools inférieurs en C₁-C₄ et plus particulièrement le 2-butanol.

La température de réaction est en général comprise entre 45 et 150°C et la durée de réaction varie de préférence de 4 à 8 heures.

Selon le procédé de l'invention, le produit final obtenu en fin de réaction peut être ensuite purifié par simple lavage et/ou simple extraction à base d'un solvant choisi par exemple parmi l'heptane, un alcool inférieur en C₁-C₄ tel que le méthanol ou leurs mélanges.

Les composés de formule (I) tels que définis précédemment peuvent être utilisés dans des compositions cosmétiques ou dermatologiques en particulier dans des produits capillaires tels que des shampooings, des après-shampooings ou des lotions pour le soin des cheveux. Ils sont utilisés comme agents de traitement des cheveux permettant d'améliorer l'état de surface du cheveu et de faciliter le peignage et le démêlage.

Les exemples qui suivent servent à illustrer l'invention sans toutefois présenter un caractère limitatif.

### EXEMPLES DE PREPARATION

### EXEMPLE 1 : Préparation du chlorure de [3-(2-octyl-dodécanoyloxy)-2-hydroxypropyl]-triméthyl ammonium

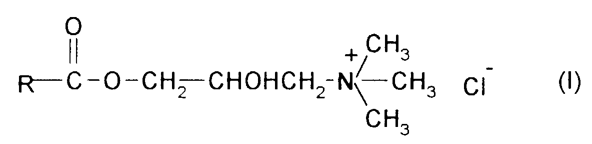

avec

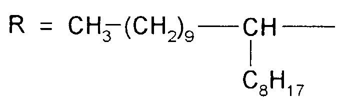

Dans un ballon muni d'une agitation, d'un thermomètre, d'une ampoule de coulée et d'un réfrigérant ascendant, on introduit :
- 100 ml de 2-butanol,
- 0,15 mole d'acide 2-octyl-dodécanoïque (46,5g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le mileu réactionnel est lavé à l'eau puis à l'heptane. La phase supérieure est concentrée à sec puis reprise dans un mélange heptane/méthanol/eau. La phase inférieure est concentrée à sec. On obtient une pâte ambrée.

| Analyses | |
|---|---|
| Indice de chlorure | 1,95 meq/g |
| Indice d'acide | 0,43 meq/g soit 19% acide (en mole) |

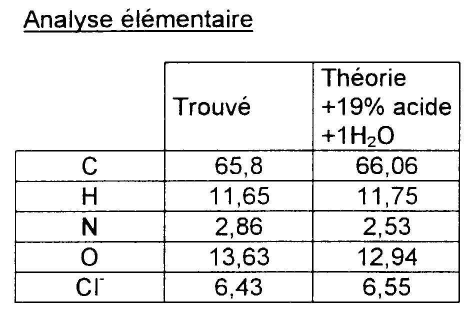

### EXEMPLE 2 : Préparation du chlorure de [3-(2-décyl-tetradécanoyloxy)-2-hydroxypropyl]-triméthyl ammonium

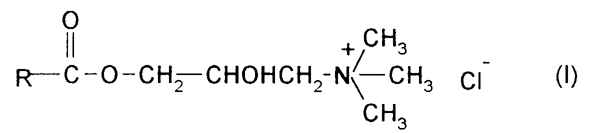

avec

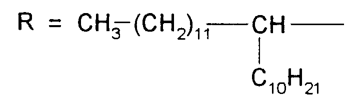

Dans un ballon muni d'une agitation, d'un thermomètre, d'une ampoule de coulée et d'un réfrigérant ascendant, on introduit:
- 100 ml de 2-butanol,
- 0,15 mole d'acide 2-décyl-tetradécanoïque (36,8g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le milieu réactionnel est lavé à l'eau puis à l'heptane. La phase supérieure est concentrée à sec puis reprise dans un mélange heptane/méthanol/eau. La phase inférieure est concentrée à sec. On obtient une pâte jaune clair.

| Analyses | |
|---|---|
| Indice de chlorure | 1,71 meq/g |
| Indice d'acide | 0,174 meq/g soit 10% acide (en mole) |

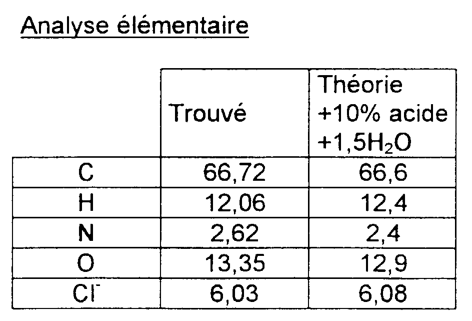

### EXEMPLE 3 : Préparation du chlorure de [3-(2-butyl-octanoyloxy)-2-hydroxypropyl]-triméthyl ammonium

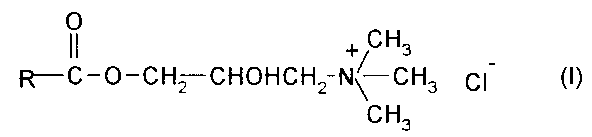

avec

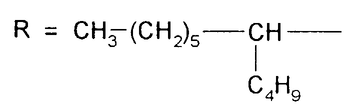

Dans un ballon muni d'une agitation, d'un thermomètre, d'une ampoule de coulée et d'un réfrigérant ascendant, on introduit:
- 100 ml de 2-butanol,
- 0,15 mole d'acide 2-butyl-octanoïque (30g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le milieu réactionnel est lavé à l'eau puis à l'heptane. La phase supérieure est concentrée à sec puis reprise dans un mélange heptane/méthanol/eau. La phase inférieure est concentrée à sec. On obtient une pâte ambrée.

| Analyses | |
|---|---|
| Indice de chlorure | 2,60 meq/g |
| Indice d'acide | 0,11 meq/g soit 4,2% acide (en mole) |

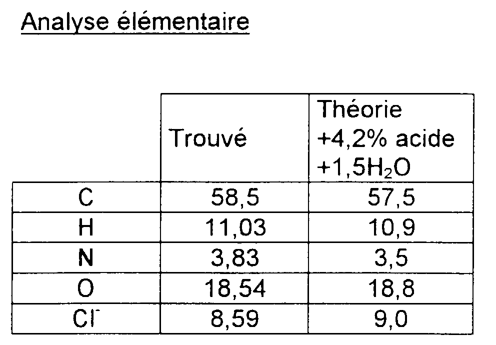

### EXEMPLE 4 : Préparation du chlorure de [3-(2-hexyl-décanoyloxy)-2-hydroxypropyl]-triméthyl ammonium

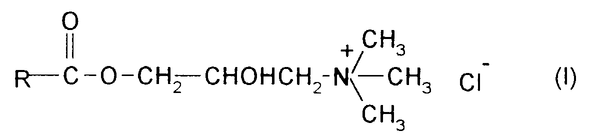

avec

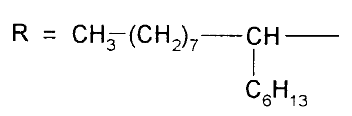

Dans un ballon muni d'une agitation, d'un thermomètre, d'une ampoule de coulée et d'un réfrigérant ascendant, on introduit :
- 100 ml de 2-butanol,
- 0,15 mole d'acide 2-hexyl-décanoïque (38,4g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le milieu réactionnel est lavé à l'eau puis à l'heptane. La phase supérieure est concentrée à sec puis reprise dans un mélange heptane/méthanol/eau. La phase inférieure est concentrée à sec. On obtient une pâte ambrée.

| Analyses | |
|---|---|
| Indice de chlorure | 1,98 meq/g |
| Indice d'acide | 0,066 meq/g soit 3,3% acide (en mole) |

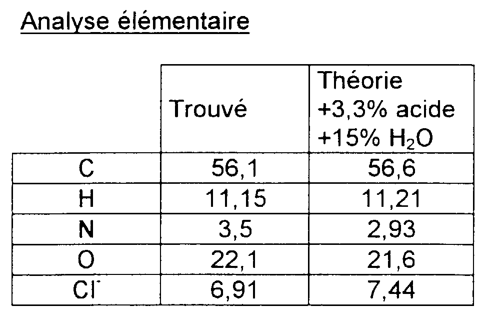

## Revendications

1. Procédé de préparation d'ammoniums quaternaires hydroxypropylés à fonction ester d'acide gras de formule (I) suivante : 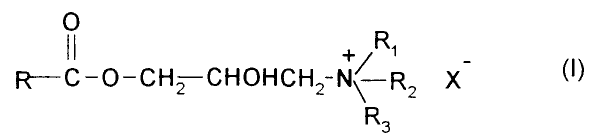 dans laquelle
R désigne une chaîne alkyle, ramifiée ou linéaire, saturée ou insaturée en C₇-C₃₅ ;
R₁, R₂ et R₃, identiques ou différents, désignent une chaîne alkyle, ramifiée ou linéaire, saturée ou insaturée en C₁-C₁₈ ;
X⁻ désigne un halogénure ou un anion choisi dans le groupe constitué par : 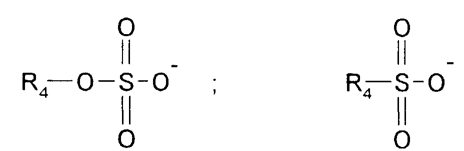 et 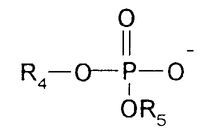 dans lesquels R₄ et R₅, identiques ou différents, désignent une chaîne alkyle, ramifiée ou linéaire, saturée ou insaturée en C₁-C₈ ;caractérisé par le fait qu'il consiste en une seule étape à faire réagir, dans un solvant alcoolique à reflux, un sel d'acide carboxylique aliphatique gras RCOOH ou un acide carboxylique aliphatique en catalyse basique avec un composé de formule (Il) suivante : 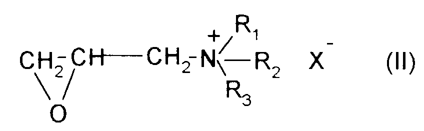 où R, R₁, R₂, R₃ et X⁻ ont les mêmes significations indiquées ci-dessus.

2. Procédé selon la revendication 1, selon lequel on prépare les composés de formule(I) où R₁, R₂ et R₃ désignent simultanément méthyle et X⁻ désigne l'ion chlorure.

3. Procédé selon la revendication 1 ou 2, caractérisé par le fait que le sel de l'acide RCOOH est un sel de métal alcalin ou alcalino-terreux.

4. Procédé selon l'une quelconque des revendications 1 à 3, le solvant alcoolique utilisé est choisi parmi les alcools inférieurs en C₁-C₄

5. Procédé selon la revendication 4, dans lequel le solvant alcoolique utilisé est choisi le 2-butanol.

6. Procédé selon l'une quelconque des revendications 1 à 5, selon lequel la température de réaction est comprise entre 45 et 150°C et la durée de réaction varie de 4 à 8 heures.

7. Procédé selon l'une quelconque des revendications 1 à 6, selon lequel le produit final obtenu en fin de réaction est ensuite purifié par simple lavage et/ou simple extraction à base d'un solvant choisi parmi l'heptane, un alcool inférieur en C₁-C₄ ou leurs mélanges.
